# EUROPÄISCHE PATENTSCHRIFT

(11) **EP 2 252 695 B1**
(45) Veröffentlichungstag und Bekanntmachung des Hinweises auf die Patenterteilung: **11.11.2015**
(21) Anmeldenummer: 09715546.9
(22) Anmeldetag: 23.02.2009
(51) Int. Cl.: C12P 5/02, C12P 7/04, C12N 1/38

(54) **VERFAHREN ZUR FERMENTATION VON BIOMASSE**
METHOD FOR FERMENTING BIOMASS
PROCÉDÉ POUR LA FERMENTATION DE BIOMASSE

(30) Priorität: 25.02.2008 AT 3112008
(43) Veröffentlichungstag der Anmeldung: 24.11.2010
(73) Patentinhaber: Roth, Hermann, 65346 Eltville (DE)
(72) Erfinder: Roth, Hermann, 65346 Eltville (DE)
(74) Vertreter: Richardt Patentanwälte PartG mbB
(86) Internationale Anmeldenummer: PCT/EP2009/001272
(87) Internationale Veröffentlichungsnummer: WO 2009/106280

(56) Entgegenhaltungen:
- WO-A-02/21932
- US-A- 5 447 850
- SINGH SHALINI ET AL: "Increased biogas production using microbial stimulants" BIORESOURCE TECHNOLOGY, Bd. 78, Nr. 3, Juli 2001 (2001-07), Seiten 313-316, XP002543708 ISSN: 0960-8524
- DATABASE BIOSIS [Online] BIOSCIENCES INFORMATION SERVICE, PHILADELPHIA, PA, US; 1981, TIWARI K P ET AL: "EFFECT OF SOME ORGANIC ACIDS AND ALKALOIDS ON LACTIC-ACID FERMENTATION" XP002543711 Database accession no. PREV198172030722 & ZENTRALBLATT FUER BAKTERIOLOGIE PARASITENKUNDE INFEKTIONSKRANKHEITEN UND HYGIENE ZWEITE NATURWISSENSCHAFTLICHE ABTEILUNG MIKROBIOLOGIE DER LANDWIRTSCHAFT DER TECHNOLOGIE UND DES UMWELTSCHUTZES, Bd. 136, Nr. 1, 1981, Seiten 70-73,
- DRSATA J ET AL: "SANGUINARINE AND CHELERYTHRINE AS INHIBITORS OF AROMATIC AMINO ACID DECARBOXYLASE" JOURNAL OF ENZYME INHIBITION, NEW YORK, NY, US, Bd. 10, Nr. 4, 1. Januar 1996 (1996-01-01), Seiten 231-237, XP001064019
- KRIVJANSKY V ET AL: "Induction of respiration-deficient mutants in Saccharomyces cerevisiae by chelerythrine" FEMS MICROBIOLOGY LETTERS, BLACKWELL PUBLISHING, AMSTERDAM, NL, Bd. 120, Nr. 1-2, 1. Juli 1994 (1994-07-01), Seiten 87-91, XP023917504 ISSN: 0378-1097 [gefunden am 1994-07-01]
- COLOMBO MARIA LAURA ET AL: "Pharmacological activities of Chelidonium manjus L. (Papaveraceae)" PHARMACOLOGICAL RESEARCH, Bd. 33, Nr. 2, 1996, Seiten 127-134, XP002543709 ISSN: 1043-6618
- VIEIRA S L ET AL.: "Performance of Broilers Fed Diets Supplemented with Sanguinarine-Like Alkaloids and Organic Acids" THE JOURNAL OF APPLIED POULTRY RESEARCH, Bd. 17, Nr. 1, 2008, Seiten 128-133, XP002543710

## Beschreibung

Die Erfindung betrifft ein Verfahren zur Fermentation von Biomasse, insbesondere ein Verfahren zur Fermentation von Biomasse zur Gewinnung von Energieträgern, beispielsweise Biogas, biogenem Kraftstoff etc.

Die Erzeugung von Energie, d.h. elektrischem Strom, Biogas, wie z.B. Methan, biogenen Kraftstoffen bzw. Kraftstoffzusätzen, wie z.B. Butanol oder Ethanol, aus regenerierbaren Rohstoffen hat weltweit politisch und wirtschaftlich hohen Stellenwert und wirtschaftliche Priorität in vielen Ländern, in denen Rohstoffe aus der Agrarwirtschaft, aus der Forstwirtschaft oder aus der Abfallwirtschaft zur Verfügung stehen. In der EU, in Nordamerika sowie in Brasilien wird dem Einsatz von Biokraftstoff als Beimischkomponente zu konventionellem Kraftstoff gesetzlich Priorität eingeräumt, und der Zusatz von Ethanol oder Biodiesel zu konventionellem Kraftstoff ist gesetzlich vorgegeben. Energie aus Wind oder Sonne sowie aus der Fermentation von Biomasse und anschließender Verstromung wird in die Energiesysteme eingeschleust. Biogas kann außerdem aufgereinigt und dann in den bestehenden Distributionsnetzen für Erdgas zur Erzeugung von Wärme oder Strom oder zum Betrieb von Kraftfahrzeugen verwendet werden.

Bei der Fermentation von pflanzlichen Substraten, wie Getreide, Körnern, Gräsern, Faserstoffen, wie Holzstoffen, und deren Bausteinen, wie Eiweiß, Stärke, Stickstoff-freien Extraktstoffen, Cellulose, Fetten etc., von vergärbaren, biomassehaltigen Reststoffen, wie Klärschlamm, Biomüll oder Speiseresten, oder von tierischem Wirtschaftsdünger, wie Gülle, Dung oder Jauche, zersetzen in aerobem oder anaerobem Milieu bestimmte Mikroorganismen, wie beispielsweise Bakterien oder Pilze, und deren Enzyme diese Biomasse zu kurzkettigen Verbindungen. Weitere Mikroorganismen (z.B. Methanobacter-Bakterien) sind dann in der Lage, beispielsweise aus kurzkettigen Fett-/Carbonsäuren, wie Essigsäure, energiereiche Gase, wie Methan, oder Alkohole, wie Ethanol oder Butanol, etc. zu synthetisieren. Diese Mikroorganismen liegen im Idealfall in Reinkultur im Fermenter vor. In der Praxis sind sie jedoch immer von einer ungünstigen Bakterienflora begleitet, die die Effizienz der günstigen Mikroflora einschränkt. Manche dieser unerwünschten Keime können im ungünstigen Fall sogar die gesamte Fermentation durch Verdrängen oder Vergiften der erwünschten Flora zum Erliegen bringen und zu einem Totalausfall des Prozesses führen.

Es gibt daher Bestrebungen, durch geeignete Maßnahmen die Effizienz des Fermentationsprozesses zu verbessern. Beispielsweise kann durch Eingriffe in die Ernährung der Mikroorganismen die erwünschte Bakterienflora im Fermenter gefördert, ihre Wirkung stimuliert und die Schadwirkung und Präsenz unerwünschter Keime auf ein geringes Maß gemindert werden.

Sing S. et al., Bioresource Technology, vol. 78 (2001): 313-316, beschreibt die Verwendung von Stimulatoren in der Biogasfermentation. Als Stimulatoren werden die Produkte "Aquasan" oder "Teresan" eingesetzt. Hierbei handelt es sich um Präparate pflanzlichen Ursprungs, welche Steroidester enthalten.

Es gibt in jedem biologischen Prozess auch unerwünschte Bakterien, Hefen und Einzeller, die als Nährstoff Abbauprodukte von Aminosäuren, sogenannte biogene Amine, benötigen, die sie durch enzymatische Decarboxylierung von Aminosäuren gewinnen, wie beispielsweise Kadaverin durch Decarboxylierung von Lysin oder Indol bzw. Skatol durch Decarboxylierung von Tryptophan oder Phenylalanin. Diese Amine sind giftige Stoffe, die ebenfalls die erwünschte Fermenterbiologie negativ beeinflussen oder sogar vergiften können und so zu einer Verdrängung der im Fermenter erwünschten Mikroorganismenflora führen. Es ist also wichtig, durch geeignete Eingriffe in die Fermentation oder deren Milieu die Wirkung solcher unerwünschten Organismen zu unterbinden oder ihre Decarboxylasen durch geeignete Hemmstoffe im Fermenter zu inaktivieren und so die Entstehung der biogenen Amine bereits zu Beginn dieser Kaskade zu unterbrechen.

Fachleuten ist bekannt, dass der Abbau von Stärke im Fermenter relativ rasch vonstatten geht, insbesondere wenn es sich um Stärkekörner aus Kartoffeln oder Getreide handelt. Stärkekörner aus Mais sind demgegenüber schon deutlich langsamer fermentativ zu den kurzkettigen Fett-/Carbonsäuren abbaubar. Um einen Fermenter möglichst effizient zu bewirtschaften, muss es jedoch das Ziel sein, die Geschwindigkeit der Fermentation zu optimieren, um in der gegebenen Zeit im vorhandenen Fermentervolumen möglichst viel Biomasse in Energiestoffe zu überführen. Somit ist für die Ausnutzung der Anlage insbesondere die Geschwindigkeit der Fermentation ausschlaggebend und sollte maximiert werden.

Der Abbau von N-freien Extraktstoffen (NfE), von Faserstoffen wie Cellulose oder Rohfaser oder der Abbau von Holzstoffen wie Lignin zu energiereichen Abbauprodukten, die dann beispielsweise der Methansynthese zur Verfügung stehen, verläuft dagegen im Vergleich zum Abbau anderer Biomassebestandteile am langsamsten und bestimmt die Verweilzeit des organischen Substrats im Fermenter und damit die Auslastung der gesamten technischen Anlage.

Diese langsam abgebauten Substanzen sind jedoch insbesondere als Gerüstkohlenhydrate der bestimmende Anteil der energieliefernden Biomasse aus Stängeln und Gräsern, und somit bestimmen sie die gesamte Verweildauer des Substrats im Fermenter. Sie verlangsamen also den Fermentationsprozess und machen größere technische Volumina erforderlich, als aus biologischen Gründen nötig wäre. Andererseits entsteht etwa bei der Herstellung von Biogas gerade aus diesen Substanzen das erwünschte Zwischenprodukt Acetat, welches dann effizient in Methan umgewandelt wird. Die Abbaugeschwindigkeit dieser Gerüstsubstanzen zu Acetat ist somit in vielen Fällen der begrenzende Faktor der Gesamtausbeute eines Fermenters an Methan oder anderen energiereichen Endprodukten, wie Butanol oder Ethanol, in einer vorgegebenen Zeiteinheit.

Neben der Geschwindigkeit des Abbaus dieser Gerüstkohlenhydrate, die ein Maß für die Anlageneffizienz je Zeit ist, spielt auch der Gesamtabbau dieser Gerüstkohlehydrate, d.h. der Anteil an Gerüstkohlehydraten, der am Ende der Fermentation abgebaut ist, im Vergleich zum noch intakt vorhandenen, nicht fermentierten Rest an Gerüstsubstanzen, eine wichtige Rolle.

Daher muss durch geeignete Eingriffe in die Fermentation oder durch die Zugabe von Substanzen, welche die Fermentation der Gerüstsubstanzen stimulieren, einerseits die Geschwindigkeit des Abbaus der Gerüstkohlehydrate beschleunigt und andererseits ihr Gesamtabbau, der auch ein Maß für die Ausbeute an energiereichem Endprodukt in Bezug zum Input an Gerüstkohlehydraten ist, erhöht werden.

Die vorliegende Erfindung schlägt ein Verfahren zur Fermentation von Biomasse vor. Das Verfahren ist erfindungsgemäß dadurch gekennzeichnet, dass als Fermentationsförderer mindestens ein Isochinolinalkaloid zugesetzt wird.

Gemäß einer Ausgestaltung der Erfindung ist ein Verfahren zur Herstellung von Biogas durch Fermentation von Biomasse vorgesehen, bei dem als Fermentationsförderer mindestens ein Isochinolinalkaloid zugesetzt wird.

Gemäß einer weiteren Ausgestaltung der Erfindung ist ein Verfahren zur Herstellung von biogenem Kraftstoff oder Kraftstoffzusatz durch Fermentation von Biomasse vorgesehen, bei dem als Fermentationsförderer mindestens ein Isochinolinalkaloid zugesetzt wird.

Es ist ein Vorteil der Erfindung, dass in landwirtschaftlichen, kommunalen oder industriellen Anlagen zur Erzeugung von Biogas oder von biogenen Kraftstoffen oder Kraftstoffzusätzen durch Fermentation von Biomasse die Fermentationsabläufe beschleunigt und stabilisiert werden können.

In den vorstehend genannten Verfahren kann ferner als Fermentationsförderer eine Lebendhefe eingesetzt werden, vorzugsweise *Saccharomyces cerevisiae* NCAIM 001126. Lebendhefen wie *Saccharomyces cerevisiae* fördern in anaeroben Systemen den Abbau von Milchsäure und verhindern so einen Abfall des pH-Werts, der die Fermentation zum Erliegen bringen würde. Außerdem bauen sie den unerwünschten Sauerstoff zu Kohlendioxid um und stellen so anaerobe Zustände sicher, sodass die anaeroben Bakterien, welche die Fermentation bestimmen, gefördert werden und der erwünschte Prozess stabilisiert und angeregt wird.

Die Dosierung der Lebendhefe kann in einem weiten Bereich variieren. Beispielsweise wird unter ungünstigen, d.h. für die Lebendhefe vermehrungsfeindlichen Umständen, eine hohe Dosis verwendet. Die Dosierung liegt bevorzugt im Bereich von 1 bis 500 g je Tonne Trockensubstanz der verwendeten Biomasse.

Die Lebendhefe, z.B. *Saccharomyces cerevisiae* NCAIM 00112, kann in Pulverform, als Flüssigkeit oder als Paste vorliegen. Sie wird zeitgleich mit der Biomasse oder getrennt davon in den Gärraum gebracht.

Erfindungsgemäß sind die zur Fermentationsförderung zugesetzten Isochinolinalkaloide vorzugsweise aus Protopinalkaloiden, Benzophenanthridinalkaloiden und deren Mischungen ausgewählt. Die Alkaloide können natürlichen und/oder synthetischen Ursprungs sein. Sie können in Form von Pflanzenbestandteilen oder Pflanzenpresssaft verwendet werden. Die Alkaloide können auch als Extrakte aus Pflanzenmaterialien verwendet werden. In dem erfindungsgemäßen Verfahren verwendbare Extrakte können nach jedwedem bekannten Verfahren hergestellt werden, und es können beispielsweise wässrige und/oder alkoholische Extrakte und/oder CO₂-Extrakte eingesetzt werden. Die Alkaloide können auch in Form von Salzen verwendet werden.

Die als Fermentationsförderer verwendeten Alkaloide können beispielsweise von Papaveraceae stammen. Als Beispiele für geeignete Papaveraceae kann man *Macleaya cordata, Sanguinaria canadensis, Chelidonium majus* etc. nennen. Als Beispiele für geeignete Alkaloide kann man Sanguinarin, dessen hydroxylierte Formen, Chelerythrin und dessen hydroxylierte Formen, Chelidonin, Chelirubin, Sanguirubin, Chelilutin, Sanguilutin, Protopin, α-Allocryptopin etc. nennen.

Selbstverständlich können beliebige Mischungen von synthetischen Alkaloiden, Alkaloiden natürlichen Ursprungs, Extrakten aus Pflanzenmaterialien, Alkaloidsalzen, die Alkaloide enthaltenden Pflanzenmaterialien und die Alkaloide enthaltendem Pflanzenpresssaft verwendet werden.

Die Alkaloide können in unterschiedlichen Zubereitungen vorliegen, beispielsweise als Flüssigkeit, als pulverförmige Zubereitung, als Granulat oder als Gel. Die alkaloidhaltige Zubereitung wird der Biomasse jeweils in geeigneter Weise zugesetzt, sodass eine homogene Vermischung gewährleistet ist.

Die angewendete Alkaloidmenge ist nach unten lediglich durch die Wirksamkeit der Alkaloide begrenzt. Die Gesamtalkaloidmenge bezogen auf die Trockensubstanz der Biomasse liegt bevorzugt im Bereich von 0,1 bis 500 ppm (= 0,1 bis 500 g Alkaloide je Tonne Trockenmasse Fermentationssubstrat).

In den vorstehend genannten Verfahren kann ferner als Fermentationsförderer ein mindestens eine Polysaccharidase enthaltender Enzymkomplex eingesetzt werden. Als Beispiele für die Polysaccharidasen kann man β-Glucanase, Cellulase, Xylanase, Amylase, Glucosidase, Galactosidase, Pektinase, Chitinase, Lysozym, Alginat-Lyase, Amyloglucosidase, Arabinase, Hemicellulase etc. nennen. Der Enzymkomplex kann ferner beispielsweise Proteasen und Lipasen enthalten. Ein besonders bevorzugtes Beispiel für einen solchen Enzymkomplex enthält β-Glucanase und Cellulase sowie Proteasen und Lipasen.

Die in diesem Fermentationsförderer enthaltenden Polysaccharidasen können von verschiedenen Bakterien und Hefen produziert werden. Als Beispiel kann man Hefen der Gattung *Thermomyces lanuginosus* nennen. Die Polysaccharidasen können auch von gentechnisch modifizierten Mikroorganismen hergestellt werden.

Die Dosierung des Polysaccharidase enthaltenden Enzymkomplexes kann in einem weiten Bereich variieren. Bevorzugt liegt die Polysaccharidase-Menge im Bereich von 1 bis 500 g je Tonne Trockensubstanz der Biomasse.

Der Polysaccharidase-haltige Enzymkomplex kann in Pulverform, als Flüssigkeit oder als Paste vorliegen. Er wird zeitgleich mit der zugeführten Biomasse oder getrennt davon in den Gärraum gebracht.

Der Fermentation kann eine Kombination aus einer Lebendhefe, vorzugsweise *Saccharomyces cerevisiae* NCAIM 001126, einem mindestens eine Polysaccharidase enthaltenden Enzymkomplex und mindestens einem Protopin- und/oder Benzophenanthridinalkaloid zugesetzt werden. Ein besonders bevorzugter Enzymkomplex enthält β-Glucanase und Cellulase sowie Proteasen und Lipasen. In einer bevorzugten Ausführungsform der Fermentation von Biomasse wird die Fermentationsförderer-Kombination in einer Menge im Bereich von 1 bis 500 ppm, bezogen auf die Trockensubstanz der Biomasse, zugesetzt.

Die erfindungsgemäß verwendeten Fermentationsförderer werden vorzugsweise für Fermentationen in einem Temperaturbereich von 15 bis 80°C eingesetzt.

Die in dem erfindungsgemäßen Verfahren als Substrat verwendbare Biomasse kann aus landwirtschaftlichen Produkten bzw. gezielt angebauten Energiepflanzen, wie Getreide, Gräser, Klee, Luzerne, Silomais, Grünroggen, Rüben, Raps, Stroh, Holzstoffe etc., oder vergärbaren, biomassehaltigen Reststoffen, wie Klärschlamm, Industrieabfall, Biomüll, Speisereste etc., und tierischem Wirtschaftsdünger, wie Gülle, Dung, Jauche etc., bestehen. Es können Mischungen verschiedener Ausgangsmaterialien verwendet werden.

Das Substrat wird in einer ersten Phase des Fermentationsprozesses, die auch als Hydrolyse bezeichnet wird, aufgeschlossen. Daran schließt sich die Vergärung durch die als "Vergärer" bezeichneten Mikroorganismen, insbesondere Bakterien (z.B. fettspaltende Bakterien, acidogene Bakterien, acetogene Bakterien), die fakultativ anaerob sind, an. Die technisch relevanten Produkte dieser Vergärung sind vor allem Alkohole (z.B. Methanol, Ethanol, 1,3-Propandiol) und Säuren (z.B. Essigsäure, Milchsäure, Buttersäure, Propionsäure). Der erhaltene Alkohol, insbesondere das sogenannte Bioethanol, kann beispielsweise als Kraftstoff oder Kraftstoffzusatz verwendet werden.

An die Vergärung kann sich eine weitere Stufe anschließen, die obligat anaerob ist, und bei der aus den in einer ersten Vergärungsstufe gewonnen methanogenen Substraten mithilfe von Mikroorganismen (z.B. acetogene Bakterien, Methanbildner) als Endprodukt das Biogas, Methan, entsteht.

Das Verfahren zur Fermentation von Biomasse unter Zusatz des Fermentationsförderers gemäß der vorliegenden Erfindung kann sowohl in Anlagen mit diskontinuierlicher Beschickung (z.B. Batchverfahren oder Wechselbehälterverfahren) als auch in Anlagen mit quasikontinuierlicher oder kontinuierlicher Beschickung (z.B. Durchflussverfahren, Speicherverfahren oder kombiniertes Durchfluss-Speicher-Verfahren) sowie z.B. in Trockenvergärungsverfahren, Container-Verfahren, Pfropfenströmungsverfahren, Boxen-Fermentern etc. durchgeführt werden.

In allen Fällen liegen die Vorteile des erfindungsgemäßen Verfahrens darin, dass sowohl die Geschwindigkeit der Fermentation der Biomasse beschleunigt und so die Kapazität eines Fermenters als auch der Gesamtabbau organischer Substanz zum Fermentationsprodukt signifikant erhöht wird. Das erfindungsgemäße Verfahren führt dazu,
a) dass die Zusammensetzung der Mikoorganismenpopulation zugunsten der in der jeweiligen Fermentation erwünschten Mikoorganismenflora verbessert ist,
b) dass die Produktion an erwünschtem Acetat deutlich erhöht ist,
c) dass die Gesamtausbeute an erwünschtem Endprodukt (Biogas, wie Methan, Alkohol, wie Ethanol, Butanol, etc.) signifikant erhöht wird,
d) dass der Abbau von Aminosäuren aus dem Fermentationsmaterial zu unerwünschten fermentationsfeindlichen biogenen Aminen reduziert ist,
e) dass die im Fermenter erwünschte Bakterienflora stimuliert und die unerwünschte Bakterienflora vermindert ist,
f) dass der Fermentationsprozess gezielt beeinflusst werden kann, sodass die Milieubedingungen (pH-Wert, Salzgehalt, Nährstoffversorgung etc.) für die an der Konversion beteiligten Mikroorganismen optimiert werden,
g) dass die Geschwindigkeit der Fermentation von Stärke und Faserstoffen positiv beeinflusst und so die Kapazität und Ausnutzung des Fermenters erhöht ist,
h) dass die Gesamtausbeute an energiereichem Gas oder flüssigen Produkten je zugeführter Einheit an Substrat ansteigt,
i) dass durch die gleichmäßigere Fermentation im Verlauf der Zeitachse auch der Verlauf des pH-Werts im Fermenter stabilisiert ist und dass die Ausbeute an Produkt erhöht ist und deren Verlauf im Zeitverlauf stabiler, d.h. ohne große Schwankungen, erfolgt,
j) dass somit die Anlage zur Energieerzeugung (Dynamomaschine) auf höherer Auslastung oder/und gleichmäßiger und wirtschaftlicher betrieben werden kann,
k) dass somit der Betrieb von Biogasanlagen oder von Anlagen zur fermentativen Herstellung von flüssigen Energieträgern, wie Ethanol, Butanol etc., auch unter kritischeren Gesichtspunkten der Wirtschaftlichkeit sinnvoll erscheint.

Nachstehend wird die Erfindung anhand von Beispielen ausführlicher erläutert.

### BEISPIELE

### Beispiel 1:

### Verwendung von Isochinolinalkaloiden als Fermentationsförderer

Vom Erfinder der vorliegenden Erfindung durchgeführte Versuche zur Gewinnung von Biogas bzw. Alkoholen durch Fermentation von Biomasse in sogenannten Kolbenfermentern haben gezeigt, dass der Zusatz von Isochinolinalkaloiden, insbesondere Protopin- und/oder Benzophenanthridinalkaloiden, zur Fermentation die Bakterienpopulation zugunsten der erwünschten Mikroorganismen stimuliert. Die Alkaloide vermindern die Produktion unerwünschter Buttersäure bildender Mikroorganismen und stabilisieren so den Fermentationsprozess, was sich wiederum positiv auf die Tätigkeit der Acetat- und Methanbildner auswirkt. Dies führt zu einem erhöhten Gesamtabbau der als Substrat eingesetzten organischen Masse zu energiereichen kurzkettigen Fett-/Carbonsäuren, die dann beispielsweise in der Biogasanlage zur Methanogenese zur Verfügung stehen. Damit ergibt sich insgesamt ein erhöhter Energieertrag.

In Abhängigkeit von der Dosis der Alkaloide wurde folgende Verbesserung des Gasertrags erzielt:

### Versuchsbedingungen:

### Inkubation im Kolbenfermenter, Thermoschrank mit Dauerrührwerk. 3 Dosierungen, 3 Wiederholungen je Dosierung. Spezifischer Gasertrag in Nm³/kg oTS (oTS = organische Trockensubstanz)

Fermentierte Biomasse: Getreide, Mais, Gülle und Viehexkremente.

Mikroorganismen: Es wurde die Mischflora aus einem optimal betriebenen Biogasreaktor verwendet, die aus ca. 200 individuellen Mikrobenstämmen, Protozoa, Bakterien, Hefen und Pilzen besteht.

Alkaloide: Sanguinarin und Chelerythrin. Gesamtkonzentration der Alkaloide in der als Fermentationsförderer eingesetzten Alkaloid-Vormischung: 3%.

Fermentationsbedingungen: anaerob, 40°C, normaler Luftdruck.

| Tage Fermentation | 1 | 5 | 10 | 15 | 20 | 25 | 30 | 35 |
|---|---|---|---|---|---|---|---|---|
| Gasertrag mit Benzophenanthridinalkaloiden | 0,05 | 0,31 | 0,52 | 0,59 | 0,63 | 0,64 | 0,65 | 0,66 |
| Gasertrag Kontrolle | 0,05 | 0,27 | 0,45 | 0,55 | 0,6 | 0,61 | 0,62 | 0,63 |
| Differenz absolut | 0 | 0,04 | 0,07 | 0,04 | 0,03 | 0,03 | 0,03 | 0,03 |
| Differenz % | 0 | 15 % | 16 % | 7 % | 5 % | 5 % | 5 % | 5 % |

Es zeigt sich, dass der Zusatz von Benzophenanthridinalkaloiden in Form einer 3%igen Vormischung mit der Dosierung der Vormischung von 1, 10 und 100 ppm jeweils den Gasertrag in den ersten 15 Tagen um 10 bis 15 % im Vergleich zum Kontrollmedium erhöht und am Tag 20 bereits der maximal wirtschaftliche Gasertrag erreicht ist. Die Kontrollgruppe erreicht diesen Gasertrag im Versuchsverlauf nie. Die Differenz zugunsten des erfindungsgemäßen alkaloidhaltigen Fermentationsförderers beträgt bis zum Versuchsende 5 %. Die Dauer des Versuchs entspricht etwa dem Zeitrahmen der Fermentation in praktischen Großfermentern. Diese Resultate sind demnach praxisrelevant und umsetzbar.

Somit kann durch den Zusatz von Benzophenanthridinalkaloiden die Gesamtausbeute an Gas zum Ende des Versuchslaufs um mindestens 5 % erhöht werden, die Ausbeute in den ersten 15 Tagen war sogar um 15 bis 16 % erhöht, was auf eine Stimulierung der Fermentationsgeschwindigkeit durch eine effizientere Bakterientätigkeit hinweist. Es kann aber auch die Verweildauer des Substrats im Fermenter um 30 % der Zeit reduziert werden, und zwar bei gleicher Ausbeute an energiereichen Abbauprodukten, wie Acetat oder Biogas oder Ethanol etc., d.h. man kann dem Fermenter im selben Zeitraum 30 % mehr an organischer Masse zuführen und erwünschten Produkten umwandeln und so die fermentierte Masse und den Ertrag an Produkten in den ersten 18 Tagen der Fermentation um 30 % erhöhen, oder man kann den Fermenter bei Neuinvestition um 30 % kleiner planen und dennoch dieselbe Menge an Biomasse zu Produkt (= energiereiches Methan) umsetzen und so die Investitionseffizienz drastisch erhöhen.

Analoge Versuche mit anderen Protopin- und/oder Benzophenanthridinalkaloiden haben vergleichbare Ergebnisse geliefert.

### Beispiel 2 (Verpleichsbeispiel):

### Verwendung eines mindestens eine Polysaccharidase enthaltenden Enzymkomplexes als Fermentationsförderer

Fermentationsprozesse sind multifunktionelle Prozesse, und es greifen unterschiedlichste Faktoren in den Prozess ein, insbesondere in den Prozess der Fermentation von Faserbestandteilen. Da die Bakterienflora des Fermenters ständigen Schwankungen hinsichtlich der Zusammensetzung, Art, Dichte im Milieu (KBE/g = koloniebildende Einheiten/g) etc. ausgesetzt ist, unterliegt ihre Produktion an Enzymen zum Abbau des organischen Substrats ebenfalls täglichen, jahreszeitlichen oder klimatischen Schwankungen. Der Fermenter kann daher aufgrund steter suboptimaler mikrobieller Produktion an faserabbauenden Enzymen nie an seiner Kapazitätsgrenze ausgelastet werden. Fehler und Schwankungen bei der Zuführung des Substrats haben ebenfalls einen großen Einfluss. Das kann die Qualität des Substrats betreffen, die Temperatur, Tageszeit, aber auch Fremdstoffe, Schadstoffe, Wassergehalt etc.

Es wird daher als Fermentationsförderer ein Enzymkomplex zum gezielten Aufschluss von Faserkomponenten zugesetzt. In Frage kommen hierzu insbesondere Enzyme, die den Abbau schwer abbaubarer Substrate, wie dies vor allem die Lignin- und Rohfaserfraktion des Substrats ist, verbessern. Dadurch kann die Gesamtausbeute an erwünschtem Gas oder energiereichen Flüssigkeiten erhöht werden, und der Fermentationsverlauf kann konstanter gemacht und auf ein effektiveres Niveau gehoben werden.

Der als Fermentationsförderer zugesetzte Enzymkomplex enthält mindestens eine Polysaccharidase. Die Verdaulichkeit und der Gesamtnutzungsgrad von Gerüstkohlehydraten, wie Cellulose, Lignin oder NfE (N-freie Extraktstoffe), wird dadurch signifikant verbessert. Da diese Kohlehydrate in Fermentationsanlagen zur Herstellung von Biogas oder biogenen Kraftstoffen üblicherweise ein wesentlicher Bestandteil der eingesetzten Biomasse sind, ist eine effiziente Nutzung dieser Faserstoffe wichtig, um derartige Anlagen wirtschaftlich zu führen und die Ausnutzung der Rohstoffe zu optimieren.

Versuche des Erfinders der vorliegenden Erfindung mit einem Fermenter zur Erzeugung von Biogas unter Verwendung eines mindestens eine Polysaccharidase enthaltenden Enzymkomplexes zeigten insofern herausragend positive Effekte, als der Gesamtabbau von Gerüstkohlehydraten und die Geschwindigkeit des Abbaus dieser Substrate signifikant verbessert wurden.

### Versuchsbedingungen:

Inkubation im Kolbenfermenter. 3 Dosierungen, 3 Wiederholungen je Dosierung. Spezifischer Gasertrag Nm³/kg oTS (oTS = organische Trockensubstanz)
Fermentierte Biomasse: Getreide, Heu, Gras, Mais, Gülle.
Mikroorganismen: Übliche Mischpopulation eines Biogasfermenters. Es wurde die Mischflora aus einem optimal betriebenen Biogasreaktor verwendet, die aus ca. 200 individuellen Mikrobenstämmen, Protozoa, Bakterien, Hefen und Pilzen besteht.
Fermentationsförderer: Der zugesetzte Enzymkomplex, der hier als Rumiferment bezeichnet werden soll, enthält β-Glucanase und Cellulase sowie Proteasen und Lipasen.
Fermentationsbedingungen: anaerob, 40°C, normaler Luftdruck.

| Tage Fermentation | 1 | 5 | 10 | 15 | 20 | 25 | 30 | 35 |
|---|---|---|---|---|---|---|---|---|
| Gasertrag mit Enzymkomplex | 0,05 | 0,3 | 0,52 | 0,6 | 0,64 | 0,65 | 0,66 | 0,66 |
| Gasertrag Kontrolle | 0,05 | 0,25 | 0,45 | 0,55 | 0,6 | 0,62 | 0,62 | 0,63 |
| Differenz absolut | 0 | 0,05 | 0,07 | 0,05 | 0,04 | 0,03 | 0,04 | 0,03 |
| Differenz % | 0 | 20 % | 16 % | 9 % | 6 % | 5 % | 6 % | 5 % |

Mit dem Zusatz des Polysaccharidase enthaltenden Enzymkomplexes konnte sowohl die Gasbildung in den ersten 15 Tagen der Fermentation positiv verändert werden als auch der gesamte Gasertrag aus Biomasse signifikant, nämlich um 5 %, erhöht werden. Die Polysaccharidase verbessert somit die Ausnutzung der Gerüstkohlehydrate, wie Cellulose oder Holzfaser, signifikant, was eine höhere Acetatbildung ergibt, die dann von den Methanbildnern in das gewünschte Biogas Methan umgewandelt werden kann.

Dies ist insbesondere in allen Anlagen von größtem wirtschaftlichen Nutzen, die als Substrate faserreiches pflanzliches Material wie Maissilage, Ganzpflanzensilage (GPS) aus Getreide, Grassilage, Rübenschnitzel, Zuckerrüben, Stroh jeder Art, Holzstoffe, Schilf, Abfallgrüngut aus Rasen- oder Grünlandschnitt etc. verwenden.

### Beispiel 3 (Vergleichsbeispiel):

### Verwendung von Lebendhefen als Fermentationsförderer am Beispiel von Saccharomyces cerevisiae NCAIM 001126

Die Fermentation von Biomasse zu kurzkettigen Fett-/Carbonsäuren, die dann der Methanogenese zur Verfügung stehen, funktioniert desto effizienter, je optimaler der pH-Wert im System ist und je besser Luftsauerstoff aus dem System ferngehalten wird.

Durch die tägliche Zufuhr an Biomasse in den Fermenter wird jedoch mit der voluminösen Biomasse und deren Durchmischung im Zufuhrmischer tagtäglich sehr viel neuer Sauerstoff in die Fermentation eingetragen, was zu Fehlgärungen und der Entwicklung von unerwünschten Keimen führen kann.

Durch die Zufuhr leicht verdaulicher Kohlehydrate wie Stärke aus Getreide oder Maissilage oder Maiskolben-Silage wird eine erhebliche Menge an Milchsäure (Lactat) eingetragen, die den pH-Wert im Fermenter drastisch senken würde und so die Fermentation zum Erliegen bringen könnte. Lebende Hefezellen fördern und stimulieren bekanntlich die Verstoffwechslung von Milchsäure durch Bakterien wie *Streptococcus bovis* zu Wasserstoff und Kohlenstoff und vermeiden so, dass die Milchsäure als stark wirkende Säure angereichert den pH-Wert im System gefährlich senkt.

Außerdem verbrauchen lebende Hefezellen Sauerstoff und können Kohlendioxid freisetzen. In einem vom Erfinder der vorliegenden Erfindung durchgeführten Versuch wurde daher ein selektierter Stamm einer Lebendhefe, nämlich *Saccharomyces cerevisiae* NCAIM 001126, zu der Fermentation zugesetzt. Es zeigte sich, dass diese Art durch ihren ständigen Sauerstoffverzehr und den durch sie geförderten Abbau der Milchsäure den pH-Wert im Fermenter stabilisieren und die Fermentation stabilisieren und verbessern kann.

### Versuchsbedingungen:

### Inkubation im Kolbenfermenter. 3 Dosierungen, 3 Wiederholungen je Dosierung. Spezifischer Gasertrag Nm³/kg oTS (oTS = organische Trockensubstanz)

Fermentierte Biomasse: wie in Beispiel 1 bzw. 2

Mikroorganismen: Es wurde die Mischflora aus einem optimal betriebenen Biogasreaktor verwendet, die aus ca. 200 individuellen Mikrobenstämmen, Protozoa, Bakterien, Hefen und Pilzen besteht.

Fermentationsbedingungen: anaerob, 40°C, normaler Luftdruck.

| Tage Fermentation | 1 | 3 | 5 | 8 | 10 |
|---|---|---|---|---|---|
| Gasertrag mit *Saccharomyces cerevisiae* | 0,05 | 0,22 | 0,30 | 0,48 | 0,50 |
| Gasertrag Kontrolle | 0,05 | 0,20 | 0,26 | 0,40 | 0,46 |
| Differenz absolut | 0 | 0,02 | 0,04 | 0,08 | 0,04 |
| Differenz % | 0 | 10 % | 15 % | 20 % | 9 % |

Der Versuch wurde in einem stationären System (Kolbenfermenter) durchgeführt, dem lediglich zu Beginn des Versuchs Sauerstoff zugeführt wurde und das anschließend sauerstoffdicht verriegelt war. Somit konnte der Verbrauch an Sauerstoff durch die Hefe direkt nach der Zufuhr der Biomasse simuliert werden und die Aktivität der Gaserzeugung gezeigt werden

Es konnte gezeigt werden, dass der speziell selektierte Stamm *Saccharomyces cerevisiae* NCAIM 001126 sowohl die Geschwindigkeit der Fermentation als auch die Gesamtausbeute an gasförmigem Produkt signifikant erhöht. Selbstverständlich kann auch jede andere Lebendhefe verwendet werden, deren charakteristische Eigenschaft der Verbrauch von Sauerstoff und/oder die Förderung des Abbaus der Milchsäure ist.

### Beispiel 4:

### Zusatz einer Kombination aus Benzophenanthridinalkaloiden + Polysaccharidase-haltigem Enzymkomplex + Saccharomyces cerevisiae NCAIM 001126

Zur Untersuchung synergistischer Effekte des Zusatzes mehrerer Fermentationsförderer wurde die folgende Kombination eingesetzt:
- Sanguinarin und Chelerythrin, Gesamtkonzentration der Alkaloide in der Alkaloid-Vormischung: 3%.
- β-Glucanase und Cellulase sowie Proteasen und Lipasen,
- *Saccharomyces cerevisiae* NCAIM 001126.

In einem Praxisbetrieb wurde folgender Versuch angelegt:

Der Fermenter war zu Versuchsbeginn gefüllt mit Standardsubstrat ohne die genannte zu evaluierende Fermentationsförderer-Kombination. Das Standardsubstrat war eine Biomassemischung aus Maissilage, Exkrementen, Grassilage, Getreide etc.

Die Fermentationsförderer-Kombination wurde in der ersten Woche höher dosiert, damit der Fermenter (2-mal je 1206 Kubikmeter Faulraum) rasch eine erwünschte Konzentration der gewünschten Produkte je Kubikmeter erreicht.

Als Dosierung der jeweiligen Produkte wurde jene gewählt, die in den Versuchen im Kolbenfermenter die jeweils besten Resultate erbracht hatte. Es waren dies 30 g der Alkaloid-Vormischung je Tonne Trockenmasse, 50 g des Enzymkomplexes und 50 g des Hefestamms *Saccharomyces cerevisiae* NCAIM 001126. Nach einer Zeit von 8 Wochen wurde das Mischungsverhältnis nach ökonomischen Gesichtspunkten optimiert.

Die Anlage hatte eine hervorragende Leistung. So liegt die Energieerzeugung aus Biomasse je Tonne Trockenmasse der Biomasse um 30 % über dem Sollwert nach technischen Vorgaben und auf einem maximalen Niveau von 98 bis 99 % Auslastung der elektrischen Dynamomaschine. Somit war hier keine weitere Steigerung durch die erfindungsgemäß verwendete Kombination zu erwarten. Da jedoch die Kosten für die Biomasse über 80 % der Anlagenkosten ausmachen, wäre es ökonomisch wünschenswert, wenn mit weniger Input an Biomasse immer noch dieselbe Menge an Gas beziehungsweise elektrischem Strom erzeugt werden könnte.

### Beispiel 5:

### Versuchsverfahren in der Praxis auf einer 500-KWel-Biogasanlage:

Biogasanlage:
   Gärbehälter 2 x 1206 m³
Typus: Durchflussverfahren
1 Nachgärbehälter 2280 m³
Elektrischer Dynamo: 2 x 250 KWel
Temperaturbereich: mesophil, 43°C

**Substrateinsatz zu Beginn:**

| Substrat | Frischmasse [in t] | Trockenmasse [in %] |
|---|---|---|
| Maissilage | 21 | 30 |
| Rindergülle | 1,4 | 28 |
| Getreide Triticale, geschrotet | 0,5 | 86 |

### Fermentationsförderer:

Eine Kombination von 50 g der im Beispiel 4 verwendeten Alkaloid-Vormischung, 50 g des im Beispiel 4 verwendeten Enzymkomplexes und 30 g des Hefestamms *Saccharomyces cerevisiae* NCAIM 001126 je Tonne Trockenmasse der zu fermentierenden Biomasse.

### Anwendung:

1 Woche hohe Dosierung als Vorlaufzeit: 1,5 g der Versuchsmischung je KW el. Leistung und Tag
1 Woche Adaptationszeit: 1,5 g
12 Wochen Versuchszeit: 1 g der Versuchsmischung je KW el. Leistung und Tag

Die verwendete Kombination brachte in der schon vorher optimal laufenden Anlage folgendes Resultat:
Die Versuchsmischung wurde ab dem 3. Monat eingesetzt. Der 2. Monat diente als Vergleichsgröße zur Bewertung.

| Kilowatt el. Energie eingespeist pro Tag | | | | | |
|---|---|---|---|---|---|
| | Soll KTBL | Ist | Mehrertrag | davon Erfindung | Acetat |
| Aus zugeführterBiomasse | | | | | mg/l |
| Monat 1, Tag 1 | 8500 | 10900 | 1400 | nicht eingesetzt | 1500 |
| Monat 1, Tag 15 | 8500 | 10000 | 1500 | nicht eingesetzt | 1400 |
| Monat 2, Tag 1 | 8500 | 10900 | 2400 | nicht eingesetzt | 1500 |
| Monat 2 | 8200 | 10700 | 2500 | nicht eingesetzt | 1500 |

| Beginn des Einsatzes der Versuchsmischung | | | | | |
|---|---|---|---|---|---|
| Monat 3, 1. Hälfte | 8200 | 11500 | 3300 | + 700 | 1300 |
| Monat 3, 2. Hälfte | 8000 | 11000 | 3000 | + 400 | 1500 |
| Monat 4, 1. Hälfte | 8000 | 11000 | 3000 | + 400 | 1800 |
| Monat 4, 2. Hälfte | 8000 | 11500 | 3500 | + 900 | 2000 |

Die Tabelle zeigt, dass die Versuchsanlage eine exzellente Leistung bietet, die zu Beginn des Versuchs um 32 % über der Leistung vergleichbarer Anlagen für dieselbe Masse an Biomasse liegt. Mit Hilfe der verwendeten Kombination ist es möglich, diese Mehrleistung sogar noch auszudehnen auf 38 % Mehrleistung (3000 bis 3500 KW/d) im Vergleich zum KTBL(Kuratorium für Technik und Bauwesen in der Landwirtschaft)-Standardbetrieb.

Die Fermentation von Biomasse zur Erzeugung von Biogas in Form von Methan ist stark abhängig von einer ausreichenden Produktion von Acetat, das aus der Fermentation der Biomasse durch acetatbildende Bakterien entsteht. Dabei spielt aber auch der pH-Wert des gesamten Systems eine wesentliche Rolle.

Durch die verwendete Kombination konnte die Produktion an Acetat, nachdem die Mikroorganismenflora an diese Kombination adaptiert war, 20 bis 30 % erhöht werden, was wiederum die Grundvoraussetzung für eine höhere Methanogenese ist.

Der Zusatz der verwendeten Kombination, bestehend aus einer Mischung von Benzophenanthridinalkaloiden + Polysaccharidase-haltigem Enzymkomplex + *Saccharomyces cerevisiae* NCAIM 001126, zur Fermentation von Biomasse hat den Vorteil, dass
- die Geschwindigkeit der Fermentation der Biomasse zu energiereichen Gasen oder Flüssigkeiten gerade in den ersten Tagen der Fermentation gefördert wird,
- der Zeitbedarf für eine wirtschaftliche Ausnutzung der Biomasse um bis zu 30 % reduziert wird,
- der wesentliche Fermentationsparameter Acetat um 20 bis 30 % erhöht wird,
- die Ausnutzung der organisch gebundenen Kohlehydrate verbessert wird,
- für dieselbe Leistung ca. 5 % Biomasse eingespart werden können,
- aus demselben Input an Biomasse 8 bis 10 % mehr nutzbare Energie (Biogas, wie Methan, Elektrizität, biogene Kraftstoffe, wie Alkohole, Wärme etc.) gewonnen werden können,
- die Wirtschaftlichkeit von Biofermentationsanlagen verbessert wird,
- ein hoher Beitrag zur Entschärfung der Konkurrenz um die Verwendung der Biomasse zwischen Nahrungsproduktion und Energieproduktion geleistet werden kann.

Zu den Biogasanlagen sind sowohl Anlagen zu zählen, die nach geltendem EEG (Erneuerbare-Energien-Gesetz) betrieben werden, als auch Anlagen, die nicht nach geltendem EEG betrieben werden. Daneben kann das erfindungsgemäße Verfahren auch zur großtechnischen Herstellung biogener Kraftstoffe bzw. Kraftstoffzusätze, wie Ethanol, Butanol oder andere mit derselben Zielstellung, eingesetzt werden.

Neben dem Einsatz in Anlagen zur Erzeugung von Biogas bzw. biogenen Kraftstoffen/Kraftstoffzusätzen kann das erfindungsgemäße Verfahren auch in Abwasserreinigungsanlagen sowie in Abfallbehandlungsanlagen angewendet werden.

## Patentansprüche

1. Verfahren zur Gewinnung von Energieträgern aus Biomasse in einem Fermentationsprozess, **dadurch gekennzeichnet, dass** zur Förderung der Fermentation mindestens ein Isochinolinalkaloid zugesetzt wird.

2. Verfahren nach Anspruch 1, **dadurch gekennzeichnet, dass** die Isochinolinalkaloide aus Protopinalkaloiden, Benzophenanthridinalkaloiden und deren Mischungen ausgewählt sind.

3. Verfahren nach Anspruch 1 oder 2, **dadurch gekennzeichnet, dass** die Alkaloide natürlichen und/oder synthetischen Ursprungs sind.

4. Verfahren einem der Ansprüche 1 bis 3, **dadurch gekennzeichnet, dass** die Alkaloide in Form von Pflanzenbestandteilen, Pflanzenpresssaft, Extrakten aus Pflanzenmaterialien, in Form ihrer Salze, als synthetische Alkaloide oder Mischungen davon zugesetzt werden.

5. Verfahren einem der Ansprüche 1 bis 4, **dadurch gekennzeichnet, dass** die Alkaloide von Papaveraceae stammen.

6. Verfahren nach einem der Ansprüche 1 bis 5, **dadurch gekennzeichnet, dass** die Gesamtalkaloidmenge im Bereich von 0,1 bis 500 ppm, bezogen auf die Trockensubstanz der verwendeten Biomasse, liegt.

7. Verfahren nach einem der Ansprüche 1 bis 6, **dadurch gekennzeichnet, dass** der erhaltene Energieträger Biogas ist.

8. Verfahren nach einem der Ansprüche 1 bis 6, **dadurch gekennzeichnet, dass** der erhaltene Energieträger ein flüssiger biogener Kraftstoff oder Kraftstoffzusatz ist.

9. Verwendung mindestens eines Isochinolinalkaloids als die Fermentation fördernder Fermentationszusatz bei der Fermentation von Biomasse zur Gewinnung von Energieträgern.

10. Verwendung nach Anspruch 9, **dadurch gekennzeichnet, dass** das Isochinolinalkaloid ausgewählt ist aus Benzophenanthridinalkaloiden, Protopinalkaloiden und deren Mischungen.

## Claims

1. A method for the recovery of energy carriers from biomass in a fermentation process, **characterised in that** at least one isoquinoline alkaloid is added in order to promote the fermentation.

2. The method according to Claim 1, **characterised in that** the isoquinoline alkaloids are selected from protopine alkaloids, benzophenanthridine alkaloids, and mixtures thereof.

3. The method according to Claim 1 or 2, **characterised in that** the alkaloids are of natural and/or synthetic origin.

4. The method according to one of Claims 1 to 3, **characterised in that** the alkaloids are added in the form of plant constituents, pressed plant sap, extracts from plant materials, in the form of salts thereof, as synthetic alkaloids, or mixtures thereof.

5. The method according to one of Claims 1 to 4, **characterised in that** the alkaloids originate from Papaveraceae.

6. The method according to one of Claims 1 to 5, **characterised in that** the total alkaloid amount lies in a range from 0.1 to 500 ppm, in relation to the dry substance of the used biomass.

7. The method according to one of Claims 1 to 6, **characterised in that** the obtained energy carrier is biogas.

8. The method according to one of Claims 1 to 6, **characterised in that** the obtained energy carrier is a liquid biogenic fuel or fuel additive.

9. Use of at least one isoquinoline alkaloid as the fermentation-promoting fermentation additive in the fermentation of biomass for the recovery of energy carriers.

10. The use according to Claim 9, **characterised in that** the isoquinoline alkaloid is selected from benzophenanthridine alkaloids, protopine alkaloids, and mixtures thereof.

## Revendications

1. Procédé de production de fournisseurs d'énergie à partir de la biomasse dans un processus de fermentation, **caractérisé en ce qu'**au moins un alcaloïde d'isoquinoléine est ajouté pour la promotion de la fermentation.

2. Procédé selon la revendication 1, **caractérisé en ce que** les alcaloïdes d'isoquinoléine sont choisis parmi des alcaloïdes de protopine, des alcaloïdes de benzophénanthridine et leurs mélanges.

3. Procédé selon les revendications 1 ou 2, **caractérisé en ce que** les alcaloïdes sont d'origine naturelle et/ou synthétique.

4. Procédé selon l'une des revendications 1 à 3, **caractérisé en ce que** les alcaloïdes sont ajoutés sous la forme de constituants de plantes, de jus de plantes pressées, d'extraits de produits à base de plantes, sous forme de sels, en tant qu'alcaloïdes synthétiques ou en tant que leurs mélanges.

5. Procédé selon l'une des revendications 1 à 4, **caractérisé en ce que** les alcaloïdes sont issus de papavéracées.

6. Procédé selon l'une des revendications 1 à 5, **caractérisé en ce que** la quantité totale d'alcaloïde se situe dans le domaine de 0,1 à 500 ppm, par rapport à la substance sèche de la biomasse utilisée.

7. Procédé selon l'une des revendications 1 à 6, **caractérisé en ce que** le fournisseur d'énergie obtenu est du biogaz.

8. Procédé selon l'une des revendications 1 à 6, **caractérisé en ce que** le fournisseur d'énergie obtenu est un carburant biogène ou un additif de carburant.

9. Utilisation d'au moins un alcaloïde d'isoquinoléine en tant qu'additif améliorant la fermentation de biomasse pour la production de fournisseurs d'énergie.

10. Utilisation selon la revendication 9, **caractérisée en ce que** l'alcaloïde d'isoquinoléine est choisi parmi des alcaloïdes de benzophénanthridine, des alcaloïdes de protopine et leurs mélanges.
